# EUROPEAN PATENT SPECIFICATION

(11) **EP 3 970 653 B1**
(45) Date of publication and mention of the grant of the patent: **29.11.2023**
(21) Application number: 20382831.4
(22) Date of filing: 21.09.2020
(51) Int. Cl.: A61B 90/70, A61M 27/00, A61B 18/00, A61N 7/00, A61M 25/00

(54) **DEVICE FOR NON-INVASIVE PREVENTIVE CLEANING OF CEREBROSPINAL FLUID SHUNT SYSTEMS**
VORRICHTUNG ZUR NICHTINVASIVEN PRÄVENTIVEN REINIGUNG VON SHUNT-SYSTEMEN FÜR ZEREBROSPINALFLÜSSIGKEIT
DISPOSITIF DE NETTOYAGE PRÉVENTIF NON INVASIF DE SYSTÈMES DE DÉRIVATION DE LIQUIDE CÉPHALO-RACHIDIEN

(43) Date of publication of application: 23.03.2022
(73) Proprietor: Universidad de Sevilla, 41013 Sevilla (ES); Servicio Andaluz de Salud, 41071 Sevilla (ES)
(72) Inventor: GÓMEZ GONZÁLEZ, Emilio, 41013 Sevilla (ES); PERALES ESTEVE, Manuel A., 41013 Sevilla (ES); GUERRERO CLARO, Manuel, 41013 Sevilla (ES); MUÑOZ GONZÁLEZ, Francisco J., 41013 Sevilla (ES); MARQUEZ RIVAS, Javier, 41071 Sevilla (ES); MAYORGA BUIZA, María José, 41071 Sevilla (ES); FERNÁNDEZ LIZARANZU, Isabel, 41071 Sevilla (ES)
(74) Representative: Pons

(56) References cited:
- WO-A1-2019/150372
- US-A1- 2004 230 117
- US-A1- 2006 235 349
- US-A1- 2013 158 464
- US-A1- 2016 279 449
- Anonymous: "La UE financiará un dispositivo para paliar sintomas de la hidrocefalia", , 23 May 2019 (2019-05-23), XP055783060, Retrieved from the Internet: URL:https://www.aulamagna.com.es/cern-con- acento-andaluz-investigadores-sevillanos-c ontra-la-hidrocefalia/ [retrieved on 2021-03-08]
- Anonymous: "Combined optical imaging and ultrasound focusing for hand-held, non-invasive cleaning of implanted cerebrospinal fluid shunting devices in patients of hydrocephalus: initial design and proof-of-concept (FUSCLEAN) - ATTRACT Project", , 23 May 2019 (2019-05-23), XP055783023, Retrieved from the Internet: URL:https://attract-eu.com/showroom/projec t/combined-optical-imaging-and-ultrasound- focusing-for-hand-held-non-invasive-cleani ng-of-implanted-cerebrospinal-fluid-shunti ng-devices-in-patients-of-hydrocephalus-in itial-design-and-proof-of-concep/ [retrieved on 2021-03-08]
- Gómez-González Emilio ET AL: "Public deliverable for the ATTRACT Final Conference Combined optical imaging and ultrasound focusing for hand-held, non-invasive cleaning of implanted cerebrospinal fluid shunting devices in patients of hydrocephalus: initial design and proof-of-concept (Project FUSCLEAN)", , 23 May 2019 (2019-05-23), XP055783042, Retrieved from the Internet: URL:https://attract-eu.com/wp-content/uplo ads/2019/05/FUSCLEAN.pdf [retrieved on 2021-03-08]

## Description

### OBJECT OF THE INVENTION

The object of the invention is a device a for preventive, non-invasive, easy-to-implement, and cost-effective acoustic cleaning of cerebrospinal fluid (CSF) shunt systems (valves and catheters) implanted in patients of hydrocephalus using focused ultrasound (sonic cleaning). It is based on optical and thermal image-guided ultrasound focusing for removal of accumulated debris, clearing obstructions and improving cerebrospinal fluid flow through implanted catheters and valves.

### BACKGROUND OF THE INVENTION

Hydrocephalus is a relevant neurosurgical pathology, both in children and in the aging population. It comprises a wide set of ailments associated with alterations of the cerebrospinal fluid hydrodynamics and the biomechanical properties of the nervous central system structures.

Certain types of hydrocephalus are relatively common both in children and in the aging population. In adults, the most common is the idiopathic normal-pressure. It has an estimated incidence range of 1.8-2.2 cases/100.000 adult individuals (0.41% of the population over 65 years-old). In children, prevalence of congenital and pediatric hydrocephalus ranges to 0.5-0.8 cases/1.000 newborn.

In many cases, these pathologies are characterized by an enlargement of the lateral ventricles of the brain (due to accumulation of fluid) and by the physiological and cognitive consequences of compression of adjacent structures, damages in surrounding tissue and other effects of increased intracranial pressure.

In the majority of cases (70%), treatment usually requires neurosurgical placement of a shunt to drain CSF from brain ventricles (proximal segment, 14-20 cm), through a small hole in the skull, to a valve under the skin (cranial vault) and an outflow catheter (up to 1 m) to a distal cavity (peritoneal, heart, pleura).

Complications of shunting are frequent, and their clinical consequences define a life-threatening medical condition. They range to a 50% failure rate 2 years after implantation in pediatric patients or to 84.5% of patients requiring 1 or more shunt revisions, and 4.7% requiring 10 or more in a 20-year follow-up.

The most common complication is occlusion (particularly of proximal catheter, 27%) due to build-up of excess proteins, clots and infection. However, symptoms of occlusion are initially similar to common diseases and early diagnosis and treatment of an obstructed catheter or shunt malfunction is a defying area.

Therefore, complications are difficult to anticipate and require immediate surgical removal because of risks of serious neurological damage and even death. Such complications have a deep social impact in the quality of life of patients, their families and caregivers, and a high economic cost. When a patient experiments a CSF drainage blockage, standard (emergency) treatment is based on the neurosurgical replacement of the shunting system.

It is known from the state of the art an existing technology of "focused ultrasound surgery", which is based on the concentration of ultrasound energy to produce an intended temperature increase. However, this technology requires large, heavy and costly surgical and medical imaging equipment.

In conclusion, early diagnosis and treatment of an obstructed catheter or shunt malfunction remains as a defying area and there is no preventive technology or protocol to avoid them.

Anonymous document: *"*La UE financiará un dispositivo para paliar sintomas de la hidrocefalia", 23 May 2019 (2019-05-23), XP055783060. Retrieved from the Internet: URL:https://www.aulamagna.com.es/cern-con-acento-andaluzinvestigadores-sevillanos-contra-la-hidrocefalia/ [retrieved on 2021-03-08] describes a device that allows the non-invasive removal of obstructions affecting the valves implanted in the brains of hydrocephalus patients.

Anonymous document: *"*Combined optical imaging and ultrasound focusing for hand-held, non-invasive cleaning of implanted cerebrospinal fluid shunting devices in patients of hydrocephalus: initial design and proof-of-concept (FUSCLEAN)- ATTRACT Project", 23 May 2019 (2019-05-23), XP055783023. Retrieved from the Internet: URL: https://attract-eu. com/showroom/project/combined-opticalimaging-and-ultrasound-focusing-for-hand-held-non-invasivecleaning-of-implanted-cerebrospinal-fluid-shunting-devices-inpatients-of-hydrocephalus-initial-design-and-proof-of-concep/ [retrieved on 2021-03-08] describes a hand-held device for the application of image-guided, focused ultrasound, for preventive, non-invasive, easy-to-implement, and cost-effective acoustic cleaning of implanted shunts in patients of hydrocephalus.

Document from Gómez-González Emilio ET AL: "Public deliverable for the ATTRACT Final Conference Combined optical imaging and ultrasound focusing for hand-held, non-invasive cleaning of implanted cerebrospinal fluid shunting devices in patients of hydrocephalus: initial design and proof-of-concept (Project FUSCLEAN)", 23 May 2019 (2019-05-23), XP055783042, Retrieved from the Internet: International application No. PCT/EP2021 /075911 URL:https://attract-eu.com/wp-content/uploads/2019/05/ FUSCLEAN. pdf [retrieved on 2021-03-08] describes a hand-held device for preventive, non-invasive, easy to implement, and cost-effective acoustic cleaning of implanted shunts.

Document US2006/235349A1 describes a cerebrospinal fluid shunt (implantable devices for diversion of excess fluid from the brain to other body cavities) used to treat hydrocephalus often malfunction. A common etiology of shunt malfunction is obstruction of the distal catheter tip by accumulating particulate matter such as fat or proteinaceous debris. The proposed implantable device maintains the patency of the cerebrospinal fluid shunt with mechanical energy which serves to "scrub" the catheter lumen of particulate debris. The proposed device accomplishes this by housing a source of mechanical energy which is coupled to the external aspect of the catheter, itself traversing through a bore in the device. The energy source secondarily induces a waveform in the cerebrospinal fluid flowing through the catheter. The fluid waveform exerts shearing forces on the catheter wall and serves to disrupt the formation and accumulation of debris that potentially could occlude the shunt catheter, thereby maintaining patency of the shunt.

Document US2004/230117A1 describes an ultrasonic, sonic or vibratory energy, delivered non-invasively, minimally invasively or invasively (e.g., surgically), that is utilized to provide direct cleaning action at or to the location of the implanted device such as a prosthetic heart valve with undesirable deposits of at least some amount thereon or therein. Such ultra-sound energy may be aided by the use of a drug in association or cooperation with the acoustic irradiation. The "cleaning" acoustic energy may optionally be delivered under the guidance of an imaging modality and may be delivered in a timed or gated manner such that the valve occluders or leaflets are in a preferred position (assuming they are functioning) during exposures.

Document US2016/279449A1 described sonic sonothrombolysis systems to produce two acoustic pressure levels of insonation during stroke therapy, mid/high acoustic pressure insonation directed to the site of a blood clot where microbubbles are present to induce microbubble-mediated blood clot lysis, and low acoustic insonation directed to the region surrounding the site of the blood clot where microbubbles are present to stimulate microvascular reperfusion of the surrounding tissue. The systems simultaneously produce blood clot lysis at the site of an occlusion and stimulate reperfusion of tissue affected by the occlusion.

Document US2013/158464A1 describes a self-cleaning inlet head for use on a shunt. The head has a tube with openings disposed in predetermined positions in its wall, and a cleaning element installed inside the tube. The cleaning element may comprise a central shaft with a number of bristles protruding therefrom, preferably in locations substantially identical to the positions of the openings in the wall of the tube. Mutual vibratory motion between the cleaning element and the tube causes at least some of the bristles to enter the openings, thereby keeping them clear, and preventing tissue growth into them. The vibratory motion may be generated by the action of an external field on a responsive part of the cleaning element, such as an external magnetic field operating on a magnetic or magnetized part of the cleaning element or the bristles. Alternatively, the external field may be an ultrasound field operating on the bristles.

Document WO2019/150372A1 describes a self-cleaning catheter system for fluid passage including a catheter, configured to be implanted in a body cavity of a subject and including at least one aperture fluidly coupling the catheter to the outside thereof, a cleaning unit configured for motion in the catheter such as to at least one of mechanically prevent, remove and mitigate occlusion in the at least one aperture, and an implantable controller. The cleaning unit is functionally associated with the controller, which is configured to (i) receive at least one signal indicative of a state of occlusion in the catheter, and (ii) provide an indication of the state of occlusion at least if the at least one signal indicates a blockage in the catheter and/or (iii) activate the cleaning unit if the at least one signal indicates a blockage of the catheter.

### DESCRIPTION OF THE INVENTION

The invention is defined in claim 1. Further embodiments are defined in the dependent claims. No surgical methods form part of the invention. The present disclosure describes a hand-held device for the application of image-guided, focused ultrasound, for preventive, non-invasive, easy-to-implement, and cost-effective acoustic cleaning of implanted shunts in patients of hydrocephalus. It can be used on a routine basis to any patient.

The device is based on the potential of mechanical and thermal effects of cavitation and acoustic streaming for removal of accumulated debris, clearing obstructions and improving cerebrospinal fluid flow through implanted catheters and valves.

Image guidance and monitoring are achieved by the combination of optical (visible, near infrared and thermal infrared) and medical ultrasound imaging. These technologies complement each other and their combination will allow for a fast and precise location of the shunt and catheters, located only a few millimeters under the skin (over the skull bone).

Ultrasound focusing is achieved by the overlapping of beams emitted by individual transducers (ultrasound emitters). Focusing and concentration at the location of interest (valve and catheters) can be achieved by any of the following procedures or their combination: i) location of the transducers according to a geometrical set-up equivalent to an array of emitters from a concave surface, ii) combination of individual transducers and acoustic lenses (appropriate for the frequency of emission), and iii) stimulation of individual emitters following an appropriate phase pattern. The use of acoustic lenses and appropriate phase patterns for stimulation of emitters allows for obtaining directional focusing while keeping the transducers on a flat, planar surface.

The volumetric region of space in which the ultrasound beams concentrate is called "concentration volume" or "sonication volume".

The emitters are modelled as dipolar sources or as a vibrant surface. The device achieves a desired level of amplitude (measured in Pascals, Pa) and power density (measured in watts per square meter, W/m2) of the ultrasound field at the location of the implanted catheters and valve (sonication volume) while preserving the environment of increased temperature.

Specifically, the device comprises:
- a set of emitters, preferably a set of piezoelectric ultrasound transducers;
- an ultrasound generator of control and stimulation signals connected to the emitters, that excites the emission of ultrasound with user-selectable cycles and amplitudes; - a container for a suitable fluid (e.g. gel used for medical ultrasound imaging, water, ...) for matching ultrasound propagation from the emitters to the skin (impedance matching). The emitters are positioned inside the container, which is intended to be placed over the head of the patient, centered in the location of the valve.

Additionally, the device can comprise an optical imaging unit, which in turn, comprises:
- one or more cameras, for capturing images in the visible range (VIR), near infrared images (NIR) and thermal infrared images (TIR); and a user-controlled module for selection, overlapping and visualization of the images.
- an optically transparent viewfinder or similar optical device, with a cross-hair or similar reference mark, referenced to the sonication volume, so that the user can also aim the sonication volume using the cross-hair mark. Using the optically transparent viewfinder, the device can also be positioned over the desired location of the valve (if it can be visualized) without using the cameras.

The optical imaging unit with the cameras is used to monitor and control the process, including locating and positioning the device over the valve, and monitoring the temperature in the area of application of the ultrasound.

The application (sonication) parameters can be adjusted to the specific features and clinical status of individual patients and their shunting systems, in the current paradigm of Personalized Medicine.

In an aspect of the invention, the device also comprises a simulation module, which stores data related to the arrangement and features of the emitters and of the fluid container, and the features of the valve (a library of the different types of valves).

The simulation module can be connected to and external device, like a computer or a tablet, and perform 3D simulations of the concentration of the ultrasound beams generated by the emitters, to make sure that the proper level of acoustic energy (resulting amplitude (measured in Pa) and power density (W/m2) is achieved at the location of the valve and of the catheter.

Moreover, is also object of the present disclosure a non-claimed method, which steps can be performed in the simulation module (8), and that can be executed before the cleaning with the device on the patient.

A personalized 3D study and simulation is performed, which comprises the following steps:
- Generating a geometric model, preferably of the area where the valve is implanted, the implanted valve and the catheters, the emitters, and the frame,
- definition of material properties, to simulate for example thermal behavior, densities, sound velocities, impedances and attenuation coefficients to simulate acoustic behavior,
- creation of a mesh of elements to calculate a final solution,
- assignment of boundary conditions,
- calculation and representation of results,
- if a problem is detected in the calculation:
   ∘ adjustments and/or by means of artificial intelligence algorithms,
   ∘ the process is repeated until the problems are solved,
- if problems are detected in achieving the objective (no concentration is achieved in the valve area, or there is excessive concentration in undesirable areas):
   ∘ manual adjustments and/or by means of artificial intelligence algorithms,
- the process is repeated until the objectives are achieved.

Another aspect of the disclosure relates to computer-readable storage means comprising program instructions capable of making a computer perform the steps of the method.

The disclosure also extends to computer programs adapted so that any processing means can carry out the method. Such programs may take the form of source code, object code, an intermediate source of code, and object code, for example, as in partially compiled form, or in any other form suitable for use in implementing the processes. Computer programs also encompass cloud applications based on this procedure.

In particular, the disclosure encompasses computer programs arranged on or within a carrier. The carrier can be any entity or device capable of supporting the program. When the program is incorporated in a signal that can be directly transported by a cable or other device or medium, the carrier may be made up of said cable or another device or medium. As a variant, the carrier could be an integrated circuit in which the program is included, the integrated circuit being adapted to execute, or to be used in the execution of, the corresponding processes.

For example, the programs could be embedded in a storage medium, such as a ROM, a CD ROM or a semiconductor ROM, a USB memory, or a magnetic recording medium, for example, a floppy disk or a disk. Lasted. Alternatively, the programs could be supported on a transmittable carrier signal. For example, it could be an electrical or optical signal that could be transported through an electrical or optical cable, by radio or by any other means.

The disclosure also extends to computer programs adapted so that any processing means can carry out the method. Such programs may take the form of source code, object code, an intermediate source of code, and object code, for example, as in partially compiled form, or in any other form suitable for use in implementing the processes.

Computer programs also encompass cloud applications based on this procedure.

Therefore, another aspect of the disclosure relates to a computer-readable storage medium comprising program instructions capable of causing a computer to carry out the steps of any of the methods.

Another aspect of the disclosure relates to a transmittable signal comprising program instructions capable of causing a computer to carry out the steps of any of the methods.

The present invention presents an innovative approach to a relevant, unsolved problem in neurosurgery and healthcare: the prevention and management of a very common complication, shunt obstruction, in patients of hydrocephalus along their life. It proposes a non-invasive, easy-to-implement, and cost-effective device for preventive or therapeutic cleaning of implanted devices (valves and catheters). It implies a significant change in the management of this pathology, with a substantial impact in the quality of life of the patients and a strong reduction of costs.

The proposed solution would allow for the establishment of a new clinical, innocuous, procedure that could be performed on a routine basis in any patient to reduce the number and frequency of shunt revisions in (emergency) neurosurgical procedures. A scheduled "cleaning" treatment could be particularly useful in the first months after shunt placement, when risks of obstruction are considerably higher. This procedure could also be adapted to remove clots or other products in shunts of patients presented at emergency department with acute malfunction.

In addition, the proposed device could be extended for similar cleaning and maintenance protocols of other types of implanted devices (catheters, valves, pumps) in different clinical areas such as systems for pain control and medication delivery or fluid drainage in neurosurgery and neurology, oncology (chemotherapy), cardiology, lung and possible others.

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figure 1.- Shows a general schematic view of the device cleaning the shunt system of a patient.
Figure 2.- Shows and embodiment of the device, wherein it comprises three emitters oriented to the shunt system.
Figure 3.- Shows a scheme representation of the connections in the device.
Figure 4.- Shows a view of the emitters inside the container for the fluid.
Figure 5.- Shows an upper view of the device.
Figure 6.- Shows a view of the fastening means of the device.

### PREFERRED EMBODIMENT OF THE INVENTION

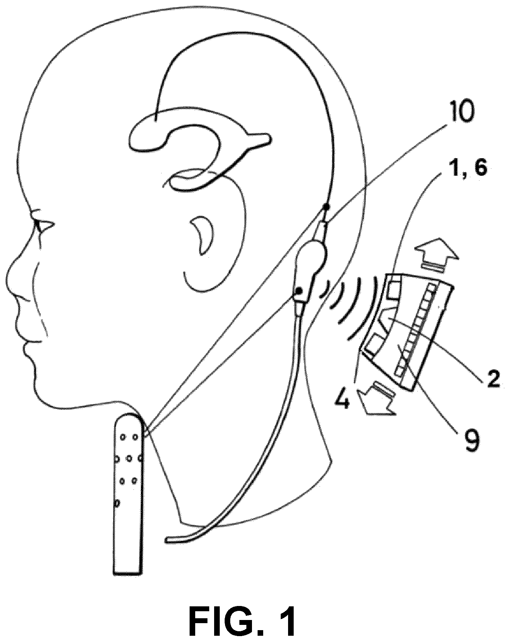

With help of figures 1 to 6 a preferred embodiment of a device for non-invasive preventive cleaning of spinal fluid shunt systems in patients with an implanted shunt (10), for example a valve and a catheter, is described below.

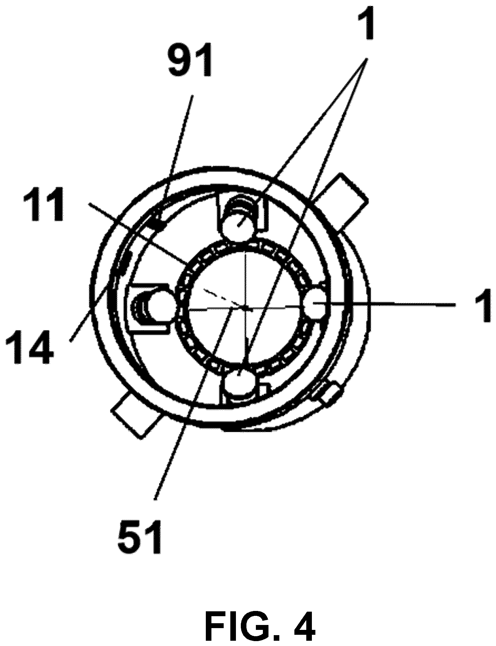

As shown in figures 1 and 4, the device comprises a frame (4) wherein a set of emitters are positioned. The number of emitters can be adjusted according to a selected geometrical set-up. A preferred array of emitters is that composed of four piezoelectric ultrasound transducers (1), located in a squared array such that their beams are emitted pointing to an imaginary axis (21) orthogonal to a plane containing the geometrical centers of the transducers (1).

This layout also requires symmetry of the transducers (1) with respect to the axis (21), which is to be called the "axis of the system" or "axis of application of the ultrasound". This axis (21) is an easy-to-interpret reference to locate the sonication volume. It is important to note that, if no acoustic lens is used, concentration can only be achieved if a symmetrical number of transducers (1) is used. Moreover, the transducers (1) should preferably be located over an imaginary plane whose orthogonal axis (21) (of symmetry) be the axis (21) of the system.

To achieve concentration at a given location along that axis (21) (without the use of acoustic lenses or specific phase patterns) the transducers (1) must be tilted a certain angle so that the orthogonal directions of their emitting faces point to the desired location along the axis (21) of the system.

Another embodiment can be with any number of transducers (1) symmetrically located (with respect to the axis (21) of the system) along a circular line contained in a plane orthogonal to the desired axis (21) of the system.

The device also comprises an ultrasound generator (3) of control and stimulation signals connected to the transducers (1), that excites the emission of ultrasound with user-selectable cycles and amplitudes.

The transducers and the ultrasound generator (3) must be selected to operate at the same frequency. The value of the frequency must be in the range of from 30 kHz to 200 kHz, but other ranges in the ultrasonic (kilohertz, kHz) and megasonic (megahertz, MHz) ranges may also be useful.

The value of the frequency is related to the relative distance between the transducers (1) and the distance from their plane to the desired location of the sonication volume along the axis (21) of the system.

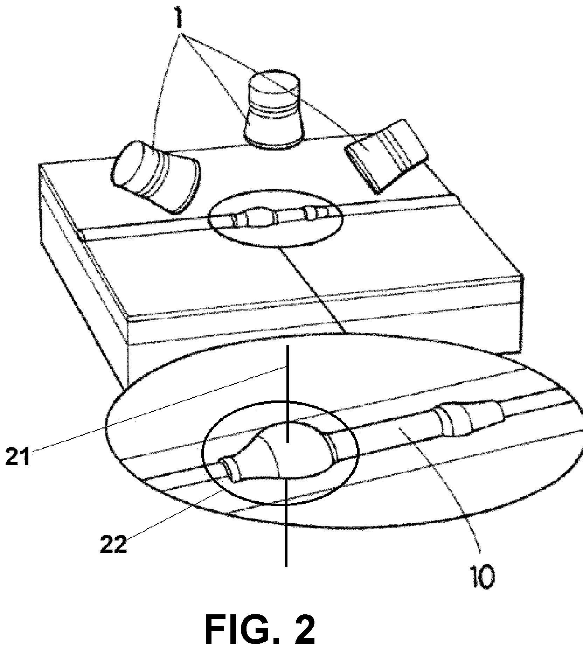

For example, using transducers (1) at 39 kHz, arranged in a symmetrical square lay-out, with side of the square of 7 cm, with simultaneous emission (no phase delay, same amplitude for all emitters), the sonication volume (22) in water is located at 9 cm measured from the face of the transducers (1) along the axis (21) of the system. Alternatively, as shown in figure 2, the transducers (1) can be arranged in a triangular lay-out, or any other lay-out that facilitates the operation of the device.

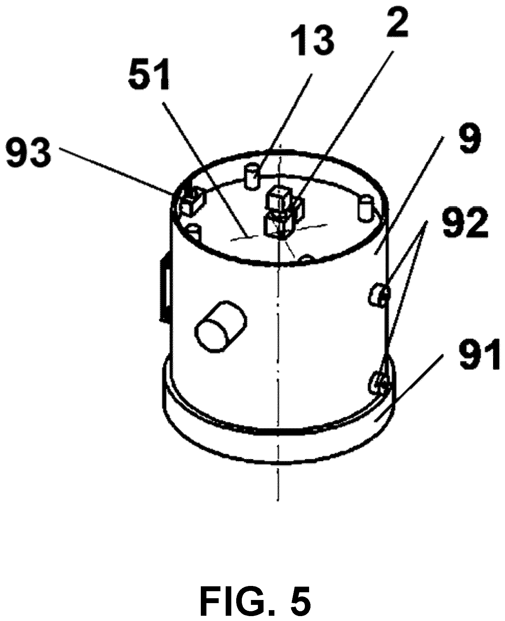

The container (9), shown in detail in figure 5, is preferably made out of an optical transparent material (e.g. methacrylate) and in a cylindrical shape. It can also adopt a truncated cone or parallelepiped shape. Inside the frame (4), the device comprises an optically transparent container (9) for a suitable fluid (e.g. gel used for medical ultrasound imaging, water, ...) for matching ultrasound propagation from the transducers (1) to the skin (impedance matching) of the patient. The transducers (1) are positioned inside the container (9) and therefore, may be partially or totally submerged in the fluid. In another embodiment, the transducers (1) may be located attached or adhered to a vibrant surface.

The container (9) can additionally comprise an optical lateral window to allow direct visualization by the user of its inner volume and a vertical graduated scale. The window can be located in the inner area of the holding platform (15) or in any other part of the lateral wall of the container (9).

The container (3) additionally comprises a vertical graduated scale, parallel to the axis (21), that allows to adjust the height of the emitters (1) with respect to the skin surface of the patient, to locate the sonication volume (22) on the targeted part of the shunt system (10). The combined use of this scale and the cross-hair mark (51) at the optical viewfinder allows for precise 3-dimensional location of the sonication volume (22) on to the desired targeted area of the shunt system (10).

The matching fluid is necessary to allow for impedance matching and, therefore, to achieve the focalization of the ultrasound beams on the desired location of the shunt system (10). However, the height of the container (9) and therefore, the volume of the fluid can be adjusted (depending on the geometry of the set-up and on the frequency of the transducers (1).

As shown in figure 1, the device can comprise a set of optional acoustic lenses (6) linked to the ultrasound transducers (1) and allowing a better focalization of the ultrasound signal. In this case, the thickness of the fluid layer and the height container (9) can be of only a few millimeters.

Alternatively, as shown schematically in figure 1, the container (9) can have an upper surface of concave shape, being the transducers (1) located on this surface and the fluid placed inside the defined volume. In this way, the concave shape of the container (9) (combined with the cylindrical walls) is the element that focalizes the ultrasound beams.

The container (9) comprises a neoprene gasket (91) placed at one end, and intended to contact and adapt to the skin of the patient without damage. It also comprises and inlet and outlet (92) that allow the movement (inflow an outflow) of the fluid through the container (9). As can be seen in figure 5, the frame (4) also comprises a vertical guiding element (93), linked to the transducers (1), to regulate the height of their plane. This element can be: grooves inside the frame (4), a rack and pinion system, or a worm screw.

The acoustic matching (impedance-matching) fluid is disposable. The perimeter of the container (9) is covered with a disposable border.

Associated to the ultrasound generator (3), the device comprises an ultrasound focalizing system (13), to control the ultrasound focalization. Several approaches can be used: a geometric control, by means of micrometric trunk, regulating the angle of inclination of the transducers (1); phase or beam steering control, by means of the ultrasound generator (3); and acoustic lens control, wherein an acoustic lens (6) is placed in front of each transducer (1) in order to focus them in the desired area.

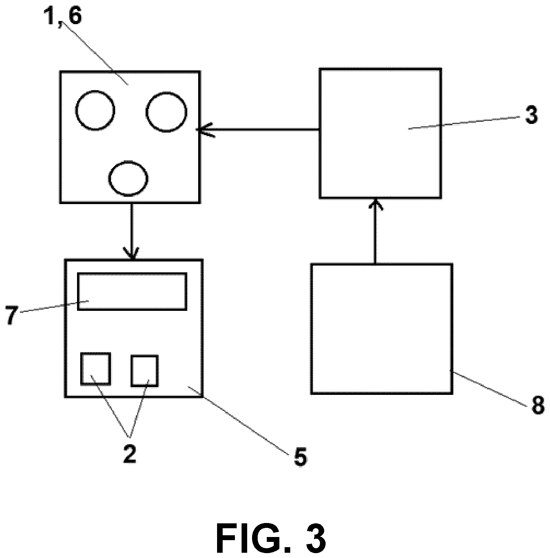

During the concentration of ultrasound beams it is important to monitor the temperature of the skin. Image guidance and monitoring is achieved by the combination of optical (visible, near infrared and thermal infrared) imaging of the shunt (10) and catheters, located only a few millimeters under the skin (over the skull bone). To this end, and connected to the holding structure of the transducers (1) the device comprises an optical imaging unit (5), shown in figure 3, which in turn comprises one or more cameras (2), for capturing images in the visible range (VIR), thermal infrared images (TIR) and near infrared images (NIR).

Moreover, the imaging unit (5) also comprises a user-controlled module (7), for example a micro-computer with a screen, for selection, overlapping and visualization of the images obtained by the cameras (2). It can additionally comprise a light beam multiplexer cube (using dichroic prisms or filters for combination of VIS / NIR / TIR images).

The optical imaging unit (5) monitors and controls the locating and positioning of the device over the shunt (10), and monitors the temperature in the area of application of the ultrasound.

As shown in figure 5, the imaging unit (5) also comprises an optical viewfinder with a cross-hair mark (51), placed on one end of the frame (4), analogous to those arranged in optical alignment and aiming systems, to adjust the device in the area to be treated.

As shown in figure 4, the device also comprises a lighting module (11) placed inside the frame (4) and oriented towards the patient. It comprises several light emitting diodes (LEDs), which can be placed in the form of one or more stripes or in the shape of a ring. The lighting module (11) also comprises a lighting controller, which allows for the regulation of the module (11) with a switch, a mobile app or an intensity regulator, being able to control the turning on and off of the module (11) as well as the intensity of the lighting.

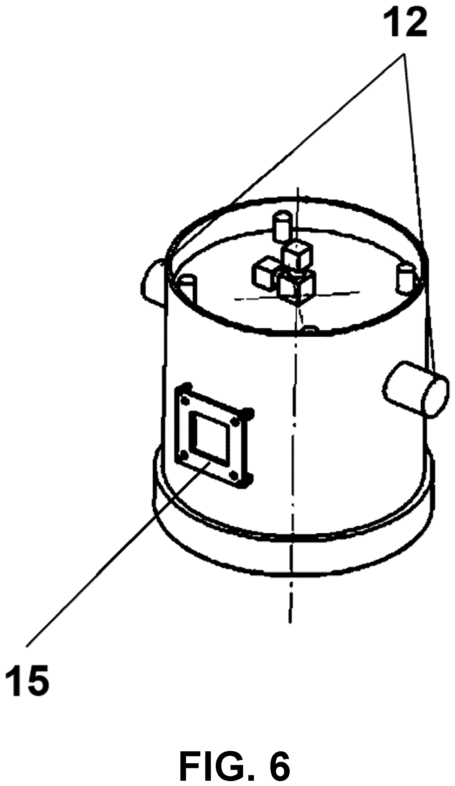

As can be seen in figure 6, the device comprises a fastening module, linked to the frame (4), which in turn comprises lateral handles (12) for the handling of the device, and a hook (15) for operating the device with standard positioning devices or robotic arms.

The device also comprises a temperature monitoring module, shown in figure 4, and linked to the frame (4) and/or the container (9), that comprises in turn a temperature sensor (thermocouple) that measures the temperature of the zone to be treated with the device, and a control module which warns if the temperature recorded by the thermocouple exceeds a certain threshold.

The device can be operated by rechargeable battery or connected to the power supply. Also, different connection methods are available: USB, Ethernet, Wi-Fi, Bluetooth or any other.

Finally, the device comprises a simulation module (8), as can be seen in figure 3, which stores data related to the arrangement and features of the transducers (1), of the fluid stored in the container (9) and of the features of the shunts (10). It can contain a library of the different types of valves.

The simulation module (8) can be connected to and external device, like a computer or a tablet, and performs 3D simulations of the concentration of the ultrasound beams generated by the transducers (1), to make sure that the proper level of acoustic energy (resulting amplitude (measured in Pa) and power density (W/m2)) is achieved at the location of the shunt (10) (valve and catheter).

The 3D simulation is a standard 3D mesh, suitable for numerical calculations using the method of finite elements (FE), and it can be used by commercial programs (e.g. COMSOL).

Specifically, is also object of the present disclosure a non-claimed method, whose steps can be performed in the simulation module (8), and that can be executed before the cleaning with the device on the patient.

A personalized 3D study and simulation is performed, which comprises the following steps:
- generating a geometric model of:
   ∘ the area where the shunt system (10) is implanted, including the thicknesses of the different layers of the patient's tissue (white and gray substances of the brain, cortical and trabecular bone of the skull, skin, scar tissue surrounding the valve, and the cerebrospinal fluid that circulates through it),
   ∘ the implanted valve and the catheters,
   ∘ the ultrasound transducers (1) (the number used), their relative positions to each other, and their distance from the valve. As well as the inclusion of additional elements for concentration such as acoustic lenses (6),
   ∘ the structure of the device (its different possible geometries), the neoprene gasket (91) with the head, and the ultrasound liquid.
- definition of material properties, such as thermal conductivities and heat capacities to simulate thermal behavior, densities, sound velocities and attenuation coefficients to simulate acoustic behavior,
- creation of a mesh of elements to calculate a final solution. The elements may have different internal functions, different geometries and different number of nodes. The mesh is optimized for the calculation to be fast and at the same time precise, being its size variable according to the proximity of the areas of interest,
- assignment of boundary conditions. At the borders of the geometric model, so that the behavior of the real system is correctly captured (no reflectance) And emission of ultrasounds in the transducers (1) at different frequencies, performing a frequency scan, and controlling the phase of the different transducers (1),
- calculation of results. Being able to use different types of resolvers (direct, iterative, ...) and different calculation modules (sound pressure in the frequency domain, in the time domain, ...),
- representation of results. Using sound intensity (W/m2) and sound pressure (Pa) variables. Spatial distribution of both fields and their amplitudes.
- If a problem is detected in the calculation (unreasonable results):
   ∘ manual adjustments are made and/or by means of artificial intelligence algorithms in the biological model, the model of the drainage system, the type of element, the boundary conditions, the optimization of the mesh, and the type of solver,
   ∘ the process is repeated until the problems are solved,
- if problems are detected in achieving the objective (no concentration is achieved in the valve area, or there is excessive concentration in undesirable areas):
   ∘ manual adjustments and/or by means of artificial intelligence algorithms are made to the model of the ultrasound emitting system, the model of the device structure, the emission of the transducers (1), and the calculation method.

The process is repeated until the objectives are achieved.

## Claims

1. Device for non-invasive preventive cleaning of a spinal fluid shunt system (10) of a patient, that comprises:
- a frame (4),
- one or more ultrasound emitters (1), with adjustable positioning, with emission intended to be oriented towards a cerebrospinal fluid shunt system (10) implanted in a patient's head, in such a way that the one or more ultrasound emitters (1) generate beams pointing to an imaginary axis (21) orthogonal to a plane containing the geometrical centers of the one or more ultrasound emitters (1),
- an ultrasound generator (3), connected to the one or more ultrasound emitters (1), that excites the emission of ultrasound from the one or more ultrasound emitters (1),
the device **characterized in that** it additionally comprises:
- a container (9) made of an optically transparent material, attached to the frame (4), intended to be placed centered over the cerebrospinal fluid shunt system (10) implanted in the patient's head,
- an acoustic-matching fluid, contained in the container (9), for matching the ultrasound propagation from the one or more ultrasound emitters (1) to the skin of the patient's head, and the ultrasound emitters (1) being placed inside the container (9), in contact with the acoustic-matching fluid.

2. The device of claim 1, wherein it additionally comprises an optical imaging unit (5), connected to the emitters (1), for monitoring skin temperature of the patient and guiding the emitters (1), that comprises one or more cameras (2) selected between a visible range camera (VIR), a thermal infrared camera (TIR) and a near infrared images (NIR).

3. The device of claim 2, wherein the optical imaging unit (5) additionally comprises an optical viewfinder with a cross-hair mark (51) for optical, visual aiming and alignment of the device with the desired location for the spinal fluid shunt system (10).

4. The device of claim 2, wherein the optical imaging unit (5) additionally comprises a user-controlled module (7) for selection, overlapping and visualization of the images obtained by the cameras (2).

5. The device according to claim 1, wherein the container (9) additionally comprises an optical lateral window to allow direct visualization by the user.

6. The device according to claim 5, wherein the container (9) additionally comprises a vertical graduated scale, parallel to the axis (21).

7. The device according to claim 1, wherein the device additionally comprises a simulation module (8), that performs 3D numerical simulations of the concentration of ultrasound beams generated by the one or more ultrasound emitters (1).

8. The device of claim 1, wherein it additionally comprises a lighting module (11) placed inside the frame (4) and oriented towards the cerebrospinal fluid shunt system (10).

9. The device of claim 1, wherein the one or more ultrasound emitters (1) are piezoelectric ultrasound transducers, and the device additionally comprises a set of optional acoustic lenses (6) linked to the ultrasound transducers.

10. The device of claim 9, wherein the piezoelectric ultrasound transducers are three arranged in a triangular lay-out.

11. The device of claim 9, wherein the piezoelectric ultrasound transducers are four arranged in a symmetrical, plane square lay-out.

12. The device according to claim 1, wherein the frame (4) additionally contains a vertical guiding element (93), linked to the ultrasound emitters (1), for precise regulation of their height.

13. The device according to claim 1, wherein it additionally comprises a fastening module, linked to the frame (4), which in turn comprises lateral handles (12) for the handling of the device, and/or a normalized holding structure (15) for operating the device with positioning devices or robotic arms.

14. The device according to claim 1, wherein the device comprises an ultrasound focalizing system (13), to control ultrasound focalization of the one or more ultrasound emitters (1).

15. The device according to claim 1, that additionally comprises a temperature monitoring module, linked to the frame (4), that comprises in turn a temperature sensor (14) and a control module which warns if the temperature recorded by the temperature sensor exceeds a certain threshold.

16. The device according to claim 1, wherein the one or more ultrasound emitters are partially submerged in the matching fluid.

17. The device according to claim 1, wherein the one or more ultrasound emitters are totally submerged in the matching fluid.

18. The device according to claim 1, wherein the container (9) additionally comprises a vibrant surface, the one or more ultrasound emitters (1) being attached to the vibrant surface.

## Patentansprüche

1. Vorrichtung zur nichtinvasiven präventiven Reinigung eines Liquor-Shunt-Systems (10) eines Patienten, die Folgendes umfasst:
- einen Rahmen (4),
- einen oder mehrere Ultraschallsender (1) mit einstellbarer Positionierung, wobei die Emission so auf ein im Kopf eines Patienten implantiertes Liquor-Shunt-System (10) ausgerichtet sein soll, dass der eine oder die mehreren Ultraschallsender (1) Strahlen erzeugen, die auf eine imaginäre Achse (21) zeigen, die orthogonal zu einer Ebene ist, die die geometrischen Mittelpunkte des einen oder der mehreren Ultraschallsender (1) enthält,
- einen Ultraschallgenerator (3), verbunden mit dem einen oder den mehreren Ultraschallsendern (1), der die Ultraschallemission von dem einen oder den mehreren Ultraschallsendern (1) anregt, wobei die Vorrichtung **dadurch gekennzeichnet ist, dass** sie zusätzlich Folgendes umfasst:
- einen aus einem lichtdurchlässigen Material hergestellten Behälter (9), befestigt am Rahmen (4), der zentriert über dem im Kopf des Patienten implantierten Liquor-Shunt-System (10) platziert werden soll,
- ein akustisch angepasstes Fluid, enthalten im Behälter (9), zur Anpassung der Ultraschallausbreitung von dem einen oder den mehreren Ultraschallsendern (1) an die Kopfhaut des Patienten, und wobei die Ultraschallsender (1) im Inneren des Behälters (9) platziert sind, im Kontakt mit dem akustisch angepassten Fluid.

2. Vorrichtung gemäß Anspruch 1, wobei sie zusätzlich eine optische Bildgebungseinheit (5), verbunden mit den Sendern (1), zur Überwachung der Hauttemperatur des Patienten und zur Führung der Sender (1) umfasst, die eine oder mehrere Kameras (2), ausgewählt aus einer Kamera im sichtbaren Bereich (VIR), eine Wärmeinfrarotkamera (TIR) und eine Nahinfrarotkamera (NIR), umfasst.

3. Vorrichtung gemäß Anspruch 2, wobei die optische Bildgebungseinheit (5) zusätzlich einen optischen Sucher mit einem Fadenkreuz (51) zum optischen, visuellen Anzielen und Ausrichten der Vorrichtung an der gewünschten Stelle für das Liquor-Shunt-System (10) umfasst.

4. Vorrichtung gemäß Anspruch 2, wobei die optische Bildgebungseinheit (5) zusätzlich ein benutzergesteuertes Modul (7) zur Auswahl, Überlagerung und Visualisierung der von den Kameras (2) erhaltenen Bilder umfasst.

5. Vorrichtung gemäß Anspruch 1, wobei der Behälter (9) zusätzlich ein optisches Seitenfenster umfasst, um eine direkte Visualisierung durch den Benutzer zu ermöglichen.

6. Vorrichtung gemäß Anspruch 5, wobei der Behälter (9) zusätzlich eine vertikale abgestufte Skala parallel zur Achse (21) umfasst.

7. Vorrichtung gemäß Anspruch 1, wobei die Vorrichtung zusätzlich ein Simulationsmodul (8) umfasst, das numerische 3D-Simulationen der Konzentration von Ultraschallstrahlen, die von dem einen oder den mehreren Ultraschallsendern (1) erzeugt werden, durchführt.

8. Vorrichtung gemäß Anspruch 1, wobei sie zusätzlich ein Beleuchtungsmodul (11) umfasst, das im Inneren des Rahmens (4) platziert und auf das Liquor-Shunt-System (10) ausgerichtet ist.

9. Vorrichtung gemäß Anspruch 1, wobei der eine oder die mehreren Ultraschallsender (1) piezoelektrische Ultraschallwandler sind und die Vorrichtung zusätzlich einen Satz optionaler akustischer Linsen (6), die mit den Ultraschallwandlern verbunden sind, umfasst.

10. Vorrichtung gemäß Anspruch 9, wobei die piezoelektrischen Ultraschallwandler drei in einem dreieckigen Grundriss angeordnete sind.

11. Vorrichtung gemäß Anspruch 9, wobei die piezoelektrischen Ultraschallwandler vier in einem symmetrischen, ebenen, quadratischen Grundriss angeordnete sind.

12. Vorrichtung gemäß Anspruch 1, wobei der Rahmen (4) zusätzlich ein mit den Ultraschallsendern (1) verbundenes vertikales Führungselement (93) zur präzisen Höhenregulierung enthält.

13. Vorrichtung gemäß Anspruch 1, wobei sie zusätzlich ein Befestigungsmodul umfasst, das mit dem Rahmen (4) verbunden ist, der wiederum seitliche Griffe (12) zur Handhabung der Vorrichtung und/oder eine normalisierte Haltestruktur (15) zur Bedienung der Vorrichtung mit Positioniervorrichtungen oder Roboterarmen umfasst.

14. Vorrichtung gemäß Anspruch 1, wobei die Vorrichtung ein Ultraschall-Fokussierungssystem (13) zur Steuerung der Ultraschallfokussierung des einen oder der mehreren Ultraschallsender (1) umfasst.

15. Vorrichtung gemäß Anspruch 1, die zusätzlich ein mit dem Rahmen (4) verbundenes Temperaturüberwachungsmodul umfasst, das wiederum einen Temperatursensor (14) und ein Steuermodul, das warnt, wenn die vom Temperatursensor erfasste Temperatur einen bestimmten Schwellenwert überschreitet, umfasst.

16. Vorrichtung gemäß Anspruch 1, wobei der eine oder die mehreren Ultraschallsender teilweise in das dazugehörige Fluid eingetaucht sind.

17. Vorrichtung gemäß Anspruch 1, wobei der eine oder die mehreren Ultraschallsender vollständig in das dazugehörige Fluid eingetaucht sind.

18. Vorrichtung gemäß Anspruch 1, wobei der Behälter (9) zusätzlich eine vibrierende Oberfläche umfasst, wobei der oder die mehreren Ultraschallsender (1) an der vibrierenden Oberfläche angebracht sind.

## Revendications

1. Dispositif pour le nettoyage préventif non invasif d'un système de dérivation de liquide céphalo-rachidien (10) d'un patient, qui comprend :
- un cadre (4),
- un ou plusieurs émetteurs d'ultrasons (1), à positionnement réglable, à émission destinée à être orientée vers un système de dérivation de liquide céphalo-rachidien (10) implanté dans la tête d'un patient, d'une telle manière que le ou les émetteurs d'ultrasons (1) génèrent des faisceaux pointant vers un axe imaginaire (21) orthogonal à un plan contenant les centres géométriques du ou des émetteurs d'ultrasons (1),
- un générateur d'ultrasons (3), connecté au ou aux émetteurs d'ultrasons (1), qui excite l'émission d'ultrasons à partir du ou des émetteurs d'ultrasons (1), le dispositif étant **caractérisé en ce qu'**il comprend de plus :
- un récipient (9) constitué d'un matériau optiquement transparent, attaché au cadre (4), destiné à être placé centré sur le système de dérivation de liquide céphalo-rachidien (10) implanté dans la tête du patient,
- un fluide d'adaptation acoustique, contenu dans le récipient (9), pour l'adaptation de la propagation d'ultrasons provenant du ou des émetteurs d'ultrasons (1) vers la peau de la tête du patient et les émetteurs d'ultrasons (1) étant placés à l'intérieur du récipient (9), en contact avec le fluide d'adaptation acoustique.

2. Dispositif selon la revendication 1, lequel comprend de plus une unité d'imagerie optique (5), connectée aux émetteurs (1), pour la surveillance de la température de la peau du patient et le guidage des émetteurs (1), qui comprend une ou plusieurs caméras (2) choisies entre une caméra dans le domaine du visible (VIR), une caméra infrarouge thermique (TIR) et des images dans le proche infrarouge (NIR).

3. Dispositif selon la revendication 2, dans lequel l'unité d'imagerie optique (5) comprend de plus un viseur optique avec une marque en croix (51) pour la visée visuelle optique et l'alignement du dispositif avec l'emplacement souhaité pour le système de dérivation de liquide céphalo-rachidien (10).

4. Dispositif selon la revendication 2, dans lequel l'unité d'imagerie optique (5) comprend de plus un module (7) commandé par l'utilisateur pour la sélection, le chevauchement et la visualisation des images obtenues par les caméras (2).

5. Dispositif selon la revendication 1, dans lequel le récipient (9) comprend de plus une fenêtre optique latérale pour permettre la visualisation directe par l'utilisateur.

6. Dispositif selon la revendication 5, dans lequel le récipient (9) comprend de plus une échelle graduée verticale, parallèle à l'axe (21).

7. Dispositif selon la revendication 1, le dispositif comprenant de plus un module de simulation (8), qui effectue des simulations numériques 3D de la concentration de faisceaux d'ultrasons générés par le ou les émetteurs d'ultrasons (1).

8. Dispositif selon la revendication 1, lequel comprend de plus un module d'éclairage (11) placé à l'intérieur du cadre (4) et orienté vers le système de dérivation de liquide céphalo-rachidien (10).

9. Dispositif selon la revendication 1, le ou les émetteurs d'ultrasons (1) étant des transducteurs à ultrasons piézoélectriques et le dispositif comprenant de plus un ensemble de lentilles acoustiques facultatives (6) reliées aux transducteurs à ultrasons.

10. Dispositif selon la revendication 9, dans lequel les transducteurs à ultrasons piézoélectriques en sont trois disposés selon un agencement triangulaire.

11. Dispositif selon la revendication 9, dans lequel les transducteurs à ultrasons piézoélectriques en sont quatre disposés selon un agencement carré plan symétrique.

12. Dispositif selon la revendication 1, dans lequel le cadre (4) contient de plus un élément de guidage vertical (93), relié aux émetteurs d'ultrasons (1), pour l'ajustement précis de leur hauteur.

13. Dispositif selon la revendication 1, lequel comprend de plus un module de fixation, relié au cadre (4), qui à son tour comprend des poignées latérales (12) pour la manipulation du dispositif et/ou une structure de tenue normalisée (15) pour le fonctionnement du dispositif avec des dispositifs de positionnement ou des bras robotisés.

14. Dispositif selon la revendication 1, le dispositif comprenant un système de focalisation d'ultrasons (13), pour commander la focalisation d'ultrasons du ou des émetteurs d'ultrasons (1).

15. Dispositif selon la revendication 1, qui comprend de plus un module de surveillance de température, relié au cadre (4), qui comprend à son tour une sonde de température (14) et un module de commande qui avertit si la température enregistrée par la sonde de température dépasse un certain seuil.

16. Dispositif selon la revendication 1, dans lequel le ou les émetteurs d'ultrasons sont partiellement immergés dans le fluide d'adaptation.

17. Dispositif selon la revendication 1, dans lequel le ou les émetteurs d'ultrasons sont totalement immergés dans le fluide d'adaptation.

18. Dispositif selon la revendication 1, dans lequel le récipient (9) comprend de plus une surface vibrante, le ou les émetteurs d'ultrasons (1) étant attachés à la surface vibrante.
